(19) Europäisches Patentamt
European Patent Office
Office européen des brevets

(11) Veröffentlichungsnummer: 0 107 839
A1

# (12) EUROPÄISCHE PATENTANMELDUNG

(21) Anmeldenummer: 83110332.0

(22) Anmeldetag: 17.10.83

(51) Int. Cl.³: C 07 C 19/02
C 07 C 21/19, C 07 C 17/16
C 07 C 17/34

(30) Priorität: 29.10.82 DE 3240110

(43) Veröffentlichungstag der Anmeldung:
09.05.84 Patentblatt 84/19

(84) Benannte Vertragsstaaten:
BE CH DE FR GB LI

(71) Anmelder: BASF Aktiengesellschaft
Carl-Bosch-Strasse 38
D-6700 Ludwigshafen(DE)

(72) Erfinder: Fischer, Martin, Dr.
Elbingerweg 1
D-6700 Ludwigshafen 29(DE)

## (54) Neue 1,1,1,4-Tetrahalogen-4-alkylpentane und Verfahren zu der Herstellung von 1,1,1,4-Tetrahalogen-4-alkylpentanen und 1,1-Dihalogen-4-alkylpentadienen-1,3.

(57) Neue 1,1,1,4-Tetrahalogen-4-alkylpentane und Verfahren zu der Herstellung von 1,1,1,4-Tetrahalogen-4-alkylpentanen und 1,1-Dihalogen-4-alkylpentadienen-1,3 durch Umsetzung von 1,1,1-Trihalogen-4-alkylpentanolen-4 mit Halogenwasserstoffsäuren bei 0 bis 130°C und gewünschtenfalls durch Umsetzung der so gebildeten 1,1,1,4-Tetrahalogen-4-alkylpentane mit Dehydrohalogenierungsmitteln bei 80 bis 170°C zu 1,1-Dihalogen-4-alkylpentadienen-1,3.

Die nach dem Verfahren der Erfindung erhaltenen 1,1,1,4-Tetrahalogen-4-alkylpentane und 1,1-Dihalogen-4-alkylpentadiene-1,3 sind Monomere und wertvolle Ausgangsstoffe für die Herstellung von Farbstoffen, Pharmazeutika und Pflanzenschutzmitteln.

EP 0 107 839 A1

# Neue 1,1,1,4-Tetrahalogen-4-alkylpentane und Verfahren zu der Herstellung von 1,1,1,4-Tetrahalogen-4-alkylpentanen und 1,1-Dihalogen-4-alkylpentadienen-1,3

Die Erfindung betrifft neue 1,1,1,4-Tetrahalogen-4-alkylpentane und Verfahren zu der Herstellung von 1,1,1,4-Tetrahalogen-4-alkylpentanen und 1,1-Dihalogen-4-alkylpentadienen-1,3 durch Umsetzung von 1,1,1-Trihalogen-4-alkylpentanolen-4 mit Halogenwasserstoffsäuren bei 0 bis 130°C und gewünschtenfalls durch Umsetzung der so gebildeten 1,1,1,4-Tetrahalogen-4-alkylpentane mit Dehydrohalogenierungsmitteln bei 80 bis 170°C zu 1,1-Dihalogen-4-alkylpentadienen-1,3.

Es sind bereits mehrere Verfahren zur Herstellung von 1,1-Dichlor-4-methylpentadien-1,3, einem Zwischenprodukt für die Synthese von Insektiziden vom Pyrethroidtyp, bekannt (Angew. Chem. 93, 719-738 (1981)). Ebenfalls ist die Umsetzung von 3-Methyl-1-buten mit Tetrachlorkohlenstoff bekannt. So kann man Tetrachlorkohlenstoff an 3-Methyl-1-buten addieren (loc.cit., Seite 721) und das so erhaltene 1,1,1,3-Tetrahalogen-4-methylpentan mit Aminen, Zinn-IV-chlorid oder Kaliumhydroxid in wasserfreien Lösungsmitteln in 1,1-Dichlor-4-methylpentadien-1,3 umwandeln. Ebenfalls kann man Chloral an Isobuten anlagern, die erhaltenen isomeren Alkohole acetylieren und diese Reaktionsprodukte mit Zink in Eisessig in ein Gemisch der isomeren 1,1-Dichlor-4-methyl-1,3 und -1,4-pentadiene umsetzen. In einer dritten Synthese wird Isobuttersäurechlorid mit 1,1-Dichlorethen in Gegenwart von Aluminiumchlorid und dann das Reaktionsprodukt mit wäßriger Natronlauge in Gegenwart von Phasentransferkatalysatoren umgesetzt; eine anschliessende Reduktion mit Natriumborhydrid und Dehydratisierung führt zum 1,1-Dichlor-4-methyl-pentadien-1,3. Weitere Synthesen gehen von Isoprenol, von Isobuten und Trichlor-

LWB/P

ethen oder von Dimethylbutenalderivaten aus (loc. cit., S. 721-722). Alle diese Verfahren sind mit Bezug auf gut zugängliche Ausgangsstoffe, einfachen, wirtschaftlichen und insbesondere großtechnischen Betrieb sowie gute Ausbeute an reinem Endstoff unbefriedigend. Nachteilig sind die Verwendung des toxischen Tetrachlorkohlenstoffs bzw. unwirtschaftlichen Borhydrids und die Entsorgung der anfallenden hohen Zinksalzmengen.

Es ist aus DE-B-25 36 504 bekannt, daß man 1,1,1-Trihalogen-4-methylpentanole-4 dehydrohalogeniert und in Gegenwart von Dehydratisierungskatalysatoren bei 50 bis 225°C zu Halogendienen dehydratisiert. In allen Beispielen wird die Herstellung von 1,1-Dichlor-4-methylpentadien-1,3 gezeigt; die Dehydrohalogenierung wird mit Kaliumhydroxid und Methanol oder Bariumchlorid-haltiger Aktivkohle bei 175°C durchgeführt. Die Dehydratisierung erfolgt mit Toluolsulfonsäure oder Eisen-III-chlorid in Toluol durch Erhitzen am Rückfluß. Ebenfalls zeigen die Beispiele eine Behandlung mit Kaliumhydrogensulfat bei 200°C oder mit 85 %iger Phosphorsäure bei 150 bis 200°C als weitere Arbeitsweisen der Dehydratisierung. Zwar werden Glykole und Glykolether als Lösungsmittel der Dehydrohalogenierung genannt, aber nur Methanol als besonders bewährt beurteilt und in den Beispielen gezeigt. Die Reihenfolge, in der Halogenwasserstoff und Wasserabspaltung vorgenommen werden, ist beliebig. Da die Wasserabspaltung durch starke Säuren oder durch Lewis-Säuren katalysiert wird, lassen sich in großtechnischem Maßstab verlängerte Reaktionszeiten nicht vermeiden, wobei ein beträchtlicher Teil des säureempfindlichen 1,3-Diens polymerisiert und die Ausbeute entsprechend abnimmt. Außerdem muß das Zwischenprodukt 1,1-Dichlor-4-methyl-4-hydroxypenten-1 durch z.B. Filtration und zwei Destillationen isoliert und gereinigt werden (Beispiel 1). Auch in der Dehydratisierungsstufe als

1. Stufe muß nach der Reaktion die organische Phase abgetrennt, mit Natriumsulfat getrocknet und im Vakuum destilliert werden, damit das Zwischenprodukt 1,1,1-Trichlor-4-methyl-penten-3 dehalogeniert werden kann (Beispiele 2 und 3).

Ein ähnliches Verfahren beschreibt die DE-A-26 16 528 in der Dehydratisierung von 1,1,1-Trihalogen-4-methylpentanol-4 zum entsprechenden 1,1,1-Trihalogen-4-methyl-3-penten und Dehydrohalogenierung des 3-Pentens mit basischen Mitteln zum 1,3-Dien. Ein Nachteil des Verfahrens besteht darin, daß bei der Wasserabspaltung, z.B. im Falle der 1,1,1-Trichlorverbindung, neben dem gewünschten 1,1,1-Trichlor-4-methylpenten-3 noch das isomere 1,1,1-Trichlor-4-methylpenten-4 entsteht. Die beiden Isomeren lassen sich mit vertretbarem

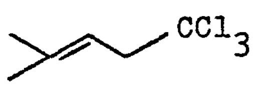

+

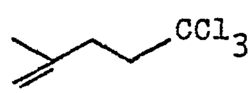

Aufwand nicht destillativ trennen. 1,1,1-Trichlor-4-methylpenten-4 wird außerdem mit Basen nicht zum gewünschten Pentadien-1,3 (Endstoff) dehydrohalogeniert. Wenn man das Gemisch der beiden Pentene in die Dehydrohalogenierung einsetzt, so erhält man ein Gemisch aus Endstoff und dem nicht umgesetzten 1,1,1-Trichlor-4-methylpenten-4, das durch Destillation ebenfalls nicht zu trennen ist. Nach dem Verfahren ist es also nicht möglich, reinen Endstoff in großtechnischem Maßstab zu produzieren.

Es ist aus EP-A-00 53 687 bekannt, daß man 1,1,1-Trichlorverbindungen mit wäßrigen Alkalilaugen in Gegenwart spezieller Mono- oder Polyole als Katalysatoren zu 1,1-Dichloralkenylenverbindungen umsetzt. Als Ausgangsstoffe können auch Trichloralkene verwendet werden. Als Substi-

tuenten von 1,1,1-Trichloralkanen bzw. 1,1,1--Trichloralkenen werden nur Hydroxygruppen, Alkyl- oder Alkoxygruppen mit 1 bis 4 Kohlenstoffatomen, jedoch nicht Halogen genannt. In den Beispielen 2 bis 4 wird gezeigt, daß man zur Herstellung von 1,1-Dichlor-4-methyl-pentadien-1,3 das schon eine Doppelbindung tragende 1,1,1-Trichlor-4-methylpenten-3 verwenden muß. Wird 1,1,1-Trichlor-4-methylpentanol-4 als Ausgangsstoff verwendet, erhält man 1,1-Dichlor--4-methylpenten-1-ol-4 als Endstoff (Beispiel 5).

Die Abspaltung von Chlorwasserstoff aus einfachen Chlorverbindungen mit basischen Mitteln ist bekannt (Houben-Weyl, Methoden der organischen Chemie, Band 5/4, Seiten 728 bis 753). Als basische Mittel werden Alkalihydroxide oder -alkoholate und tert. Amine verwendet. Die Umsetzung mit Hydroxiden führt man ebenfalls in alkoholischen Lösungsmitteln oder in Wasser durch.

Aus wirtschaftlichen Gründen ist die Verwendung von wäßrigen Alkalihydroxiden, d.h. von z.B. Natronlauge oder Kalilauge zu bevorzugen. Leider verläuft die Chlorwasserstoffabspaltung mit wäßrigen Hydroxiden häufig mit schlechter Ausbeute, erfordert sehr lange Reaktionszeiten oder bleibt auch ganz aus. Houben-Weyl weist ausdrücklich (loc.cit., Seite 744) darauf hin, daß bei Verwendung wäßriger alkalischer Lösungen Solvolyse eintritt. Man verwendet daher zur Dehydrohalogenierung rein alkoholische Lösungen, wobei lediglich als Zusatz zu solchen Lösungen, und zwar zur Zurückdrängung der Etherbildung, Triethanolamin, Alkylenglykolmonoalkylether oder Diethylenglykol verwendet werden. Besonders wirksam erscheinen Lösungen der Alkalihydroxide in organischen Lösungsmitteln allein. Die Beispiele zeigen wie spezifisch jedes Lösungsmittel je nach Zahl, Art und Stellung der Halogenatome sowie der Wasserstoffatome und weiterer Substituenten reagiert.

Es wurde nun gefunden, daß man 1,1,1,4-Tetrahalogen-4--alkylpentane der Formel

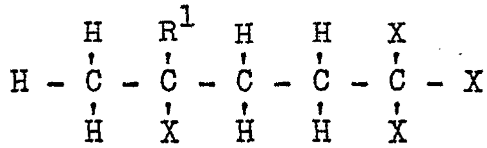

Ia

und 1,1-Dihalogen-4-alkylpentadiene-1,3 der Formel

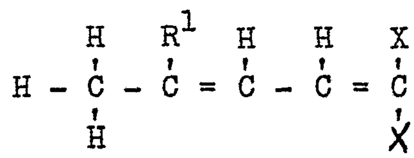

Ib,

worin $R^1$ einen Alkylrest bedeutet und die einzelnen Reste X gleich oder verschieden sein können und jeweils ein Halogenatom bezeichnen, gewünschtenfalls durch Dehydrohalogenierung von Halogenverbindungen vorteilhaft erhält, wenn man in einer ersten Stufe 1,1,1-Trihalogen-4-alkylpentanole-4 der Formel

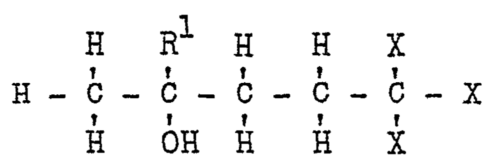

II,

worin $R^1$ und X die vorgenannten Bedeutungen besitzen, mit Halogenwasserstoffsäuren der Formel

$$H - X \qquad III,$$

worin X die vorgenannte Bedeutung besitzt, bei einer Temperatur von 0 bis 130°C umsetzt und gewünschtenfalls in einer zweiten Stufe die so erhaltenen Stoffe Ia mit Dehydrohalogenierungsmitteln bei einer Temperatur von 80 bis 170°C zu den Endstoffen Ib umsetzt.

Weiterhin wurden die neuen 1,1,1,4-Tetrahalogen-4-alkylpentane der Formel

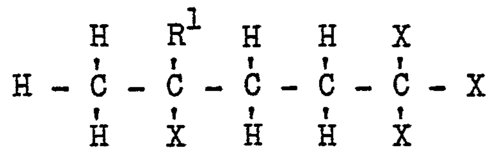

Ia,

worin $R^1$ einen Alkylrest bedeutet und die einzelnen Reste X gleich oder verschieden sein können und jeweils ein Halogenatom bezeichnen, gefunden.

Die Umsetzung wird für den Fall der Verwendung von Salzsäure, Natriumhydroxid und 1,1,1-Trichlor-4-methyl-pentanol-4 durch die folgende Formel wiedergegeben:

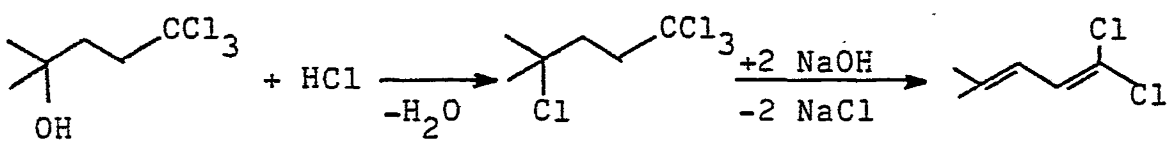


Im Vergleich zu den bekannten Verfahren liefert das Verfahren nach der Erfindung überraschend auf einfacherem und wirtschaftlicherem Wege die neuen 1,1,1,4-Tetrahalogen-4-alkylpentane und 1,1-Dihalogen-4-alkylpentadiene-1,3 in guter Ausbeute, besserer Raum-Zeit-Ausbeute und Reinheit, gerade auch im großtechnischen Maßstab. Im Hinblick auf den Stand der Technik war es überraschend, daß die bei der Umsetzung mit wäßriger Lauge genannten Schwierigkeiten nicht auftreten und vergleichsweise bessere Ausbeuten und Raum-Zeit-Ausbeuten erhalten werden. Zwar erzielt man in rein methanolischem Alkali ebenfalls gute Ausbeuten, vergleichsweise ist hier das erfindungsgemäße Verfahren einfacher, wirtschaftlicher und wegen Vermeidung von Toxizitäts- und Abwasserproblemen wesentlich umweltfreundlicher. Ein besonderer Vorteil des erfindungsgemäßen

Verfahrens besteht darin, daß der Ausgangsstoff II als Rohprodukt in Gestalt des Reaktionsproduktes seiner Herstellung eingesetzt werden kann, z.B. wie man es bei der radikalischen Addition von Chloroform an 2-Methylbuten-3-ol-2 erhält (DE-OS 26 16 528). Auch der rohe Stoff Ia braucht für seine Umsetzung in Stufe 2 nicht gereinigt zu werden.

Im Hinblick auf EP-A-00 53 687 konnte nicht erwartet werden, daß man gerade mit 4-Halogenderivaten der 1,1,1-Trihalogen-4-alkylpentane und insbesondere mit wäßrigen Alkalilaugen eine gleichzeitige Abspaltung von 2 Halogenwasserstoffmolekülen im Molekül des Ausgangsstoffs und somit eine Pentadienbildung in hoher Ausbeute erzielen würde. Es war ebenfalls überraschend, daß gerade die in der EP-A--Schrift genannten Mono- und Polyole diese hohe Pentadienausbeute liefern bzw. begünstigen, da diese Druckschrift ausschließlich die Bildung von nur einer Doppelbindung in der Alkylkette lehrt, und zur Bildung von Pentadienkörpern Alkenylverbindungen einsetzt. Im Hinblick auf diese Druckschrift auf DE-B-2 536 504 und DE-A-2 616 528 hätte man vermuten müssen, daß nur die Abspaltung eines Wasser- und eines Halogenwasserstoffmoleküls, d.h. eine Dehydrohalogenierung und Dehydratisierung in beliebiger Reihenfolge, Pentadiene in guter Ausbeute liefern. Daß bei der Dehydratisierung keine starken Säuren oder Lewis-Säuren verwendet werden müssen, sondern Hydroxyverbindungen als Katalysatoren genügen und noch dazu keine Verlängerung der Reaktionszeit und entsprechende Ausbeuteverluste durch Polymerisation des Diens mit sich bringen, war im Hinblick auf DE-B 25 36 504 überraschend. Mit Bezug auf DE-A-26 16 528 mußte auch die Bildung von Isomerengemischen und damit unreine und kaum trennbare Endstoffe erwartet werden. Daß wäßrige Alkalilaugen in Gegenwart von speziellen Hydroxyverbindungen eine hohe Ausbeute an 1,3-Dienen ohne die

Bildung teeriger Rückstände liefert, war auch im Hinblick auf die Veröffentlichung in der Angewandten Chemie (loc. cit., Seite 721, linke Spalte) überraschend. Ebenfalls hätte man im Hinblick auf die Veröffentlichung in Angewandte Chemie (loc.cit., Seite 721, rechte Spalte unten) nicht erwartet, daß die erfindungsgemäße Entfernung der Hydroxylgruppen durch Chlorsubstitution und nicht durch Dehydratisierung sowohl eine Dienbildung in der zweiten Stufe ermöglicht und gleichzeitig eine Isomerenbildung im wesentlichen vermeidet.

Der Ausgangsstoff II kann in bekannter Weise, z.B. nach der in der DE-A 2 616 528 gezeigten Arbeitsweise durch Umsetzung von Chloroform und Dimethylvinylcarbinol hergestellt werden. Bevorzugt ist die in der deutschen Patentanmeldung P 31 36 983.9 beschriebene Herstellung von 1,1,1-Trichlormethylverbindungen durch Umsetzung von Chloroform mit Olefinen in Gegenwart von Radikalstartern und von schwachbasischen Alkali- oder Erdalkalisalzen; vorteilhaft sind Umsetzungsmengen in einem Verhältnis von 2 bis 10, insbesondere 4 bis 8 Mol Chloroform je Mol Olefin. Alkali- oder Erdalkalisalze von Säuren mit Dissoziationskonstanten von höchstens $10^{-3}$, beispielsweise von Ameisensäure, Essigsäure, Propionsäure, Buttersäure, Malonsäure, Bernsteinsäure, Maleinsäure oder Benzoesäure oder basische Salze mehrbasischer Mineralsäuren wie Phosphorsäure, Borsäure, phosphorige Säure, schweflige Säure oder Kieselsäure, Alkali-, Erdalkalicarbonate und -hydrogencarbonate, bevorzugt Magnesium-, Calcium-, Lithium- und insbesondere Natrium- und Kaliumsalze; schwachbasische Salze in Mengen von 1 bis 20 Gew.%, bevorzugt 3 bis 12 Gew.%, bezogen auf Ausgangsstoff II, sind zweckmässig. Als Radikalstarter verwendet man im allgemeinen Dialkylperoxide wie Di-tert.-butylperoxide oder 2,5-Dimethyl-2,5-bis-(tert.-butylperoxi)-hexan; Perester wie

tert.-Butyl-peroxipivalat, tert.-Butylperoxioctoat, tert.--Butylperoxiisobutyrat, tert.-Butylperoximaleinsäure, 2,5-Dimethylhexan-2,5-diperbenzoat, tert.-Butylperacetat, Di-tert.-butyldiperphthalat oder tert.-Butylperbenzoat; Diacylperoxide wie Pelargonylperoxid, Decanoylperoxid, Lauroylperoxid, Propionylperoxid, Acetylperoxid, Benzoylperoxid oder p-Chlorbenzoylperoxid; Hydroperoxide wie tert.-Butylhydroperoxid, Cumolhydroperoxid oder Ketonhydroperoxide; Peroxidicarbonate wie Bis-(4-tert.-butylcyclohexyl)-peroxidicarbonat, Dicyclohexylperoxidicarbonat, Bis-(2-ethylhexyl)-peroxidicarbonat, Dibutylperoxidicarbonat, Diisopropyl-peroxidicarbonat. Man verwendet zweckmässig 1 bis 10 Mol.%, bevorzugt 3 bis 7 Mol.% Peroxid, bezogen auf das Olefin. Die Herstellung des Ausgangsstoffs II wird im allgemeinen bei einer Temperatur von 60 bis 180°C, bevorzugt von 110 bis 150°C, drucklos oder unter Druck, kontinuierlich oder diskontinuierlich durchgeführt. Der Ausgangsstoff II kann in reiner Form oder überraschend auch ohne wesentliche Reinigung, z.B. in Gestalt des Reaktionsgemisches seiner Herstellung, gegebenenfalls nach Entfernung des Lösungsmittels, eingesetzt werden.

Der Ausgangsstoff II kann mit der Halogenwasserstoffsäure in stöchiometrischer Menge oder im Überschuß, vorzugsweise in einem Verhältnis von 1 bis 10, vorzugsweise 2 bis 5 Mol Säure III je Mol Ausgangsstoff II umgesetzt werden. Bevorzugt sind 20 bis 40, vorteilhaft 30 bis 37 gew.%ige Salzsäuren. Bevorzugte Ausgangsstoffe II und III und dementsprechend bevorzugte Endstoffe Ia und Ib sind solche, in deren Formeln die einzelnen Atome X zweckmäßig gleich oder verschieden sein können und jeweils ein Bromatom oder insbesondere ein Chloratom und $R^1$ einen Alkylrest mit 1 bis 12, insbesondere 1 bis 6 Kohlenstoffatomen bezeichnen. Die vorgenannten Reste können noch durch unter den Reaktionsbe-

dingungen inerte Gruppen, z.B. Alkyl- oder Alkoxygruppen mit jeweils 1 bis 4 Kohlenstoffatomen, substituiert sein.

Es kommen beispielsweise folgende Ausgangsstoffe II in Frage: 1,1,1-Trichlor-4-hydroxy-4-methylpentan; homologe in 4-Stellung anstelle der Methylgruppe durch eine Ethyl-, Butyl-, Propyl-, Isopropyl-, Isobutyl-, sek.-Butyl, tert.--Butylgruppe substituierte 1,1,1-Trichlor-4-hydroxy-pentane; homologe 1,1,1-Tribromverbindungen.

Die Umsetzung der ersten Stufe wird bei einer Temperatur von 0 bis 130°C, vorzugsweise 30 bis 60°C, die Umsetzung der 2. Stufe im Falle saurer Dehydrohalogenierungsmittel von zweckmäßig 100 bis 170°C, im Falle basischer Katalysatoren von 80 bis 170°C, mit Unterdruck, Überdruck oder vorzugsweise drucklos, diskontinuierlich oder kontinuierlich durchgeführt. In beiden Stufen kann die Umsetzung in Anwesenheit oder Abwesenheit von unter den Reaktionsbedingungen inerten Lösungsmitteln durchgeführt werden. Bevorzugt verwendet man keine Lösungsmittel oder nur Wasser als Lösungsmittel in einer oder beiden Stufen. Man kann auch in beiden Stufen mit organischen Lösungsmitteln allein oder im Gemisch mit Wasser umsetzen. Gegebenenfalls kommen als organische Lösungsmittel in Frage: aromatische Kohlenwasserstoffe (zweckmäßig für beide Stufen), z.B. Toluol, Ethylbenzol, o-, m-, p-Xylol, Isopropylbenzol; Halogenkohlenwasserstoffe (zweckmäßig für die 1. Stufe), insbesondere Chlorkohlenwasserstoffe, z.B. Tetrachlorethylen, 1,1,2,2- oder 1,1,1,2-Tetrachlorethan, Dichlorpropan, Methylenchlorid, Dichlorbutan, Butylbromid, Chloroform, Tetrachlorkohlenstoff, 1,1,1- oder 1,1,2-Trichlorethan, Trichlorethylen, Chlorbenzol, Fluorbenzol, Brombenzol, Jodbenzol; Ether (zweckmäßig für beide Stufen), z.B. Ethylpropylether, Methyl-tert.-butylether, n-Butylethylether, Di-n-butylether, Diisobutylether, Diethylether,

Dioxan: Tetrahydrofuran (zweckmäßig für die 2. Stufe), aliphatische oder cycloaliphatische Kohlenwasserstoffe (zweckmäßig für beide Stufen), z.B. Heptan, Nonan, Benzinfraktionen innerhalb eines Siedepunktintervalls von 70 bis 190°C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan; und entsprechende Gemische. Gegebenenfalls verwendet man in einer oder in beiden Stufen das organische Lösungsmittel, zweckmäßig in einer Menge von 200 bis 10.000 Gew.%, vorzugsweise von 200 bis 1000 Gew.%, bezogen auf Ausgangsstoff II. Wasser wird im allgemeinen in einer Menge von 0,5 bis 500, insbesondere 10 bis 300 Gew.%, bezogen auf Ausgangsstoff II, verwendet, zweckmäßig zusammen mit einer Reaktionskomponente, vorteilhaft in Gestalt einer wäßrigen Halogenwasserstoffsäure, z.B. Salzsäure, oder einer wäßrigen Basenlösung, z.B. Natronlauge.

Für die Dehydrohalogenierung (Stufe 2) kommen sowohl basische als auch saure Dehydrohalogenierungsmittel in Frage; vorzugsweise werden Basen verwendet. Als saure Dehydrohalogenierungsmittel eignen sich besonders Lewis-Säuren wie Eisenchlorid, Aluminiumchlorid, Antimon-V-chlorid, Titan-IV-chlorid, Zinkchlorid oder Zinn-IV-chlorid. Die sauren Dehydrohalogenierungsmittel werden zweckmäßig in einer Menge von 1 bis 25 Gew.%, bezogen auf Ausgangsstoff II, eingesetzt. Die für die Dehydrohalogenierung üblichen Basen, wie sie beispielsweise in Houben-Weyl (loc. cit., Band V/1 b, Seiten 134-160) beschrieben sind, können in Stufe 2 verwendet werden. Die Umsetzung wird zweckmäßig mit einer basischen Verbindung in einer Menge von 2 bis 10, vorzugsweise von 3 bis 5 Äquivalenten basischer Verbindung, bezogen auf ein Mol Ausgangsstoff II, durchgeführt. Bevorzugte basische Verbindungen sind tertiäre Amine, z.B. Dialkylaniline, gegebenenfalls durch Alkyl- oder Phenyl-

gruppen substituierte Pyridine oder Chinoline, Erdalkaliverbindungen und insbesondere Alkaliverbindungen sowie entsprechende Gemische. Alkalialkoholate und insbesondere Natrium- und Kaliumhydroxid sind bevorzugt. Es kommen z.B. als basische Verbindungen in Frage: Kaliumhydroxid, Natriumhydroxid, Lithiumhydroxid, Bariumhydroxid, Natriummethylat, Natriumethylat, Natriumpropylat, Natriumisopropylat, Natriumbutylat, Natriumisobutylat, Natrium-sek.-butylat, Natrium-tert.-butylat, Natriumethylenglykolat, Trimethylamin, Triethylamin, Tripropylamin, Triisopropylamin, Tributylamin, Triisobutylamin, Tri-sek.-butylamin, Tri-tert.-butylamin, Tribenzylamin, N,N-Dimethylanilin, N,N-Diethylanilin, N,N-Dipropylanilin, N,N-Dimethyltoluidin, N,N-Diethyltoluidin, N,N-Dipropyltoluidin, Pyridin, Chinolin, 2-Methylchinolin, α-Picolin, γ-Phenylpyridin, ß-Picolin, γ-Picolin, Isochinolin.

Die Reaktion der 1. Stufe kann wie folgt durchgeführt werden: Ein Gemisch von Ausgangsstoff II und III, gegebenenfalls zusammen mit Lösungsmittel, wird in der 1. Stufe während 1 bis 10 Stunden bei der Reaktionstemperatur gehalten. Die Reaktion kann auch so durchgeführt werden, daß man so lange Halogenwasserstoff, insbesondere Chlorwasserstoff, in die Schmelze des Ausgangsstoffes II einleitet, bis das Reaktionsgemisch mit Halogenwaserstoff, insbesondere Chlorwasserstoff, gesättigt ist. Man kann die Umsetzung aber auch durch Mischen von Ausgangsstoff II mit wäßriger Salzsäure, vorteilhaft konzentrierter Salzsäure, durchführen. Aus dem Reaktionsgemisch der 1. Stufe kann der Endstoff Ia in üblicher Weise, in der Regel durch Abtrennung der wäßrigen Phase des 2-phasigen Reaktionsgemisches und Kristallisation oder Destillation, abgetrennt werden.

In einer vorteilhaften Ausführungsform wird der Stoff Ia nicht isoliert sondern in Form des Rohprodukts, z.B. in Gestalt des Reaktionsgemisches nach Abtrennung der wäßrigen Phase und gegebenenfalls des zusätzlichen organischen Lösungsmittels, als Ausgangsstoff der zweiten Stufe verwendet. In einer weiteren vorteilhaften Ausführungsform wird sowohl der Ausgangsstoff II und der Stoff Ia in vorgenannter Weise als rohes Reaktionsprodukt für die erste bzw. zweite Stufe verwendet.

Zweckmäßig kommen als Dehalogenierungsmittel und Lösungsmittel wäßrige Natron- und Kalilauge in Betracht.

In einer bevorzugten Ausführungsform wird die Umsetzung der zweiten Stufe in Gegenwart der in EP 00 53 687 beschriebenen Mono- oder Polyole als Katalysatoren durchgeführt. Vorteilhaft sind Mono- oder Polyole der Formel

$$R^3(O\overset{R^4}{\overset{|}{C}}H - \overset{R^2}{\overset{|}{C}}H)_n-OH \qquad IV,$$

in der $R^3$, $R^4$ und $R^2$ gleich oder verschieden sein können und für ein Wasserstoffatom oder einen aliphatischen Rest stehen und n eine ganze Zahl von 1 bis 10 bedeutet. Bevorzugte Katalysatoren IV sind solche, in deren Formeln $R^3$, $R^4$ und $R^2$ gleich oder verschieden sein können und für ein Wasserstoffatom oder einen Alkylrest mit 1 bis 12, insbesondere 1 bis 6 Kohlenstoffatomen stehen und n eine Zahl von 1 bis 10, insbesondere 1 bis 4 bedeutet. Die Katalysatoren IV werden zweckmäßig in Mengen von 1 bis 50 Gew.%, bevorzugt 20 bis 40 Gew.%, bezogen auf die 1,1,1-Trihalogenverbindung II, eingesetzt. Es können Katalysatorgemische verwendet werden.

Es kommen als Beispiele für Verbindungen der Formel IV in Frage:

| | |
|---|---|
| $H(OCH_2CH_2)_nOH$ | n = 2, 3, 4 |
| $CH_3(OCH_2CH_2)_nOH$ | n = 1, 2, 3, 4 |
| $C_2H_5(OCH_2CH_2)_nOH$ | n = 1, 2, 3, 4 |
| $n\text{-}C_4H_9(OCH_2CH_2)_nOH$ | n = 1, 2, 3, 4 |
| $CH_3(OCH_2-\underset{CH_3}{CH})_nOH$ | n = 1, 2 |
| $C_2H_5(OCH_2\underset{CH_3}{CH})_nOH$ | n = 1, 2 |
| $n\text{-}C_4H_9(OCH_2\underset{CH_3}{CH})_nOH$ | n = 1, 2 |

Die Reaktion der 2. Stufe kann wie folgt durchgeführt werden: Ein Gemisch von Stoff Ia, Dehydrohalogenierungsmittel, vorteilhaft Alkalihydroxid, und zweckmäßig Wasser und Katalysator wird während 0,5 bis 10 Stunden bei der Reaktionstemperatur gehalten. Dann wird aus dem Reaktionsgemisch der Endstoff in üblicher Weise, z.B. durch Waschen mit Wasser zur Entfernung des Alkalichlorids und Destillation abgetrennt. Alle Komponenten können vor der Reaktion gemischt und gemeinsam erhitzt werden. Bevorzugt erhitzt man jedoch ein Gemisch aus dem Katalysator und der 1,1,1,4-Tetrahalogenverbindung Ia auf die Reaktionstemperatur und läßt das in Wasser gelöste Alkalihydroxid in 0,5 bis 4 Stunden zulaufen. Man kann auch zusammen mit dem Katalysator das Hydroxid vorlegen und die Tetrahalogenverbindung Ia zulaufen lassen. Nach Beendigung des Zulaufs wird zweckmäßig noch 0,5 bis 9 Stunden nacherhitzt.

Die nach dem Verfahren der Erfindung erhaltenen 1,1,1,4-Tetrahalogen-4-alkylpentane und 1,1-Dihalogen-4-alkyl-pentadiene-1,3 sind Monomere und wertvolle Ausgangsstoffe für die Herstellung von Farbstoffen, Pharmazeutika und Pflanzenschutzmitteln. So können sie beispielsweise auch als Zwischenprodukte für PVC-Kunststoffe verwendet werden. Sie sind Ausgangsstoffe für die Pyrethroide Permethrin und Cypermethrin (DE-A-2 616 528, Nachr.Chem.Tech.Lab. 26, 120 bis 128 (1978)). Bezüglich der Verwendung wird auf die vorgenannten Veröffentlichungen und Ullmanns Encyklopädie der technischen Chemie, Band 13 (4. Auflage), Seiten 214-216 verwiesen. Die neuen Tetrahalogenverbindungen Ia erlauben einen neuen, wirtschaftlichen und betriebssicheren Weg zur Herstellung zahlreicher Pyrethroidverbindungen.

Beispiel 1

a) Stufe 1: 100 g 1,1,1-Trichlor-4-methyl-pentanol-4 und 200 g 35 gew.%ige Salzsäure wurden 2 Stunden bei 50°C gerührt. Nach Abtrennung der wäßrigen Phase im 2-phasigen Reaktionsgemisch erhält man 104 g 1,1,1,4-Tetrachlor-4-methylpentan vom Schmelzpunkt 48°C (95 % der Theorie), Fp. (Methanol) 54°C.

b) Stufe 2: 223 g 1,1,1,4-Tetrachlor-4-methylpentan und 70 g Triglykolmonobutylether wurden auf 100°C erhitzt. Unter Rühren ließ man 105 ml 50 gew.%ige Natronlauge in 30 Minuten zulaufen. Anschließend gab man 80 g Natriumhydroxid zu und setzte weitere 4 Stunden bei 105°C um. Nach dem Abkühlen wurde das bei der Reaktion gebildete Kochsalz in 400 ml Wasser gelöst, die wäßrige Phase abgetrennt und die organische Phase im Vakuum destilliert. Man erhielt 134 g 1,1-Dichlor-4-methyl-pentadien-1,3 (89 % der Theorie) vom Kp (30 mbar) 69°C.

Beispiel 2

a) In 100 g geschmolzenes 1,1,1-Trichlor-4-methyl-pentanol-4 wurden bei 50°C 23 g Chlorwasserstoff während 3 Stunden eingeleitet. Man beendete die HCl-Einleitung, rührte noch 1 Stunde bei 50°C nach und trennte die Wasserphase ab. Die organische Phase erstarrte bei 48°C. Man erhielt 107 g 1,1,1,4-Tetrachlor-4-methylpentan (97 % Ausbeute).

b) 100 g 1,1,1,4-Tetrachlor-4-methylpentan aus Beispiel 2a wurden mit 30 g Triglykolmonomethylether und 70 g Ätznatron 4 Stunden auf 100 bis 105°C erhitzt. Nach Aufarbeitung analog Beispiel 1b erhielt man 68,2 g 90 gew.%iges 1,1-Dichlor-4-methylpentadien-1,3 (91 % der Theorie) vom Kp. 69-70°C (30 mbar).

Beispiel 3

a) In einem Emailkessel wurden 6500 ml Chloroform und 100 g Natriumacetat unter Rühren auf 120°C erhitzt. In 4 Stunden pumpte man eine Lösung von 120 g tert.-Butylperbenzoat in 1190 g 3-Methylbuten-1-ol-3 zu und hielt anschließend das Gemisch noch 4 Stunden bei 120°C. Nach dem Abkühlen wurde das schwerlösliche Salz abfiltriert und das überschüssige Chloroform bei Normaldruck abdestilliert. Der Rückstand enthielt den Ausgangsstoff II.

b) In 100 g 70 gew.%iges, undestilliertes 1,1,1-Trichlor-4-methyl-pentanol-4 (Rückstand aus 3a) und 30 ml 35 %ige Salzsäure wurde bei 40°C während 3 Stunden 20 g Chlorwasserstoff eingeleitet. Nach Abtrennung der Wasserphase erhielt man 107 g 69 gew.%iges 1,1,1,4-

-Tetrachlor-4-methylpentan vom Schmelzpunkt 30 bis 38°C.

c) Die Umsetzung wurde analog Beispiel 2b durchgeführt. Man erhielt 46,9 g 1,1-Dichlor-4-methylpentadien-1,3 (91 % der Theorie) vom Kp. 69-70°C (30 mbar).

## Patentansprüche

1. Verfahren zur Herstellung von 1,1,1,4-Tetrahalogen-4-alkylpentanen der Formel

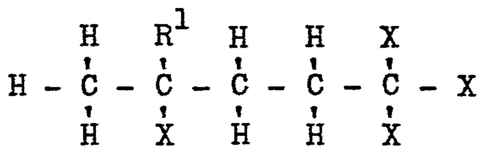 Ia

und 1,1-Dihalogen-4-alkylpentadienen-1,3 der Formel

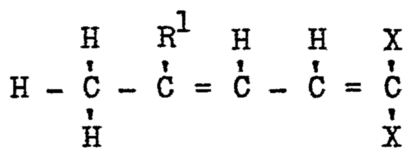 Ib,

worin $R^1$ einen Alkylrest bedeutet und die einzelnen Reste X gleich oder verschieden sein können und jeweils ein Halogenatom bezeichnen, gewünschtenfalls durch Dehydrohalogenierung von Halogenverbindungen, dadurch gekennzeichnet, daß man in einer ersten Stufe 1,1,1-Trihalogen-4-alkylpentanole-4 der Formel

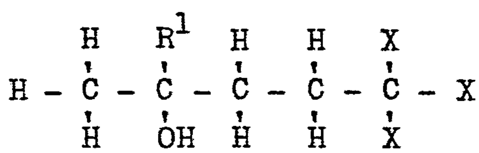 II,

worin $R^1$ und X die vorgenannten Bedeutungen besitzen, mit Halogenwasserstoffsäuren der Formel

$$H - X \qquad III,$$

worin X die vorgenannte Bedeutung besitzt, bei einer Temperatur von 0 bis 130°C umsetzt und gewünschtenfalls in einer zweiten Stufe die so erhaltenen Stoffe Ia mit Dehydrohalogenierungsmitteln bei einer Temperatur von 80 bis 170°C zu den Endstoffen Ib umsetzt.

2. 1,1,1,4-Tetrahalogen-4-alkylpentane der Formel

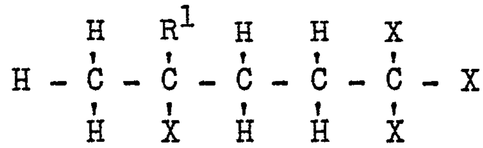

Ia

worin $R^1$ einen Alkylrest bedeutet und die einzelnen Reste X gleich oder verschieden sein können und jeweils ein Halogenatom bezeichnen.

0107839

Europäisches Patentamt

# EUROPÄISCHER RECHERCHENBERICHT

Nummer der Anmeldung

EP 83 11 0332

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| D,X | DE-A-2 616 528 (KURARAY CO.)<br>* Seite 24, Beispiel 25 *<br>--- | 2 | C 07 C 19/02<br>C 07 C 21/19<br>C 07 C 17/16<br>C 07 C 17/34 |
| A,D | DE-A-2 536 504 (BAYER)<br>--- | | |
| A | US-A-4 070 404 (LUPICHUK)<br>----- | | |
| | | | RECHERCHIERTE SACHGEBIETE (Int. Cl. 3) |
| | | | C 07 C 19/00<br>C 07 C 21/00<br>C 07 C 17/00 |

Der vorliegende Recherchenbericht wurde für alle Patentansprüche erstellt.

| Recherchenort | Abschlußdatum der Recherche | Prüfer |
|---|---|---|
| DEN HAAG | 20-01-1984 | VAN GEYT J.J.A. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82